# EUROPEAN PATENT SPECIFICATION

(11) **EP 3 517 161 B1**
(45) Date of publication and mention of the grant of the patent: **16.06.2021**
(21) Application number: 16840305.3
(22) Date of filing: 21.09.2016
(51) Int. Cl.: A61M 25/00, A61M 25/10

(54) **CATHETER AND BALLOON CATHETER**
KATHETER UND BALLONKATHETER
CATHÉTER ET CATHÉTER À BALLONNET

(43) Date of publication of application: 31.07.2019
(73) Proprietor: Asahi Intecc Co., Ltd., Seto-shi, Aichi 489-0071 (JP)
(72) Inventor: KUBO Yuta, Aichi 489-0071 (JP); OGIDO Moritaka, Aichi 489-0071 (JP); KATSURADA Takeharu, Aichi 489-0071 (JP)
(74) Representative: Winter, Brandl - Partnerschaft mbB
(86) International application number: PCT/JP2016/077894
(87) International publication number: WO 2018/055706

(56) References cited:
- JP-A- 2012 249 811
- JP-A- 2013 005 823
- JP-A- 2014 236 863
- JP-A- 2015 147 080
- JP-A- 2015 147 080
- US-A1- 2014 236 124

## Description

### TECHNICAL FIELD

The present invention relates to a catheter and balloon catheter used for diagnosing or treating a stenosis site or an obstructed segment formed inside a blood vessel or digestive organ.

### BACKGROUND ART

A stenosis site or an obstructed segment formed in a blood vessel, bile duct, pancreatic duct, and the like may result in a restricted flow of blood, bile (gall), pancreatic fluid and the like. A diagnostic or therapeutic method using a catheter is widely performed for diagnosing or treating such a stenosis site or an obstructed segment.

In general, a catheter includes a tubular inner layer, an outer layer covering the outer periphery of the inner layer, and a reinforcement layer arranged between the inner layer and the outer layer. For a catheter where the inner layer is joined with the outer layer through the reinforcement layer, enhancement of the joining strength between the inner layer and the outer inevitably becomes difficult.

As one way of solving the above problem, known is a catheter in which the joining strength between the inner layer and the outer layer is enhanced by providing a protruding portion in the inner layer, the protruding portion protruding to the outer layer side through a gap in the reinforcement layer and extending in the axis direction so as to enter into the outer layer (see Patent Document 1 below).

However, in the catheter described in Patent Document 1, the protruding portion of the inner layer only extends in one direction from the proximal end to the distal end. Therefore, disadvantageously, the outer layer may be susceptible to detachment from the inner layer when the outer layer is dragged in the direction of the distal end by a stenosis site or an obstructed segment. Further, the protruding portion of the inner layer is only provided on the outside of the reinforcement layer. Stress may be concentrated on the joining region between the protruding portion of the inner layer and the outer layer due to bending of the catheter when the catheter is inserted through a curved blood vessel, bile duct, pancreatic duct, and the like. Therefore, there remains a problem in that the outer layer may be susceptible to detachment from the inner layer.

Document US 2014/0236124 A1 discloses a catheter according to the preamble of claim 1.

### CITATION LIST

### PATENT DOCUMENT

Patent Document 1: WO2015/012185

### SUMMARY OF THE INVENTION

### TECHNICAL PROBLEM

The present invention is made in view of these circumstances. Starting from US 2014/0236124 A1, an object of the present invention is to provide a catheter and balloon catheter in which an outer layer is not easily detached from an inner layer even when the outer layer is pulled in the axis direction (the distal end and proximal end directions), and in which the risk of detachment of the outer layer from the inner layer is further reduced.

### SOLUTION TO PROBLEM

The above object can be achieved by virtue of the following means.

Embodiment 1 of the present invention provides a catheter including: a tubular inner layer; a reinforcement layer present inside the inner layer or on an outer periphery of the inner layer, the reinforcement layer having a wire wound therearound so that a gap is present between adjacent portions of the wire; and an outer layer covering the reinforcement layer, in which the inner layer has an uneven outer peripheral surface on which a protruded portion is formed in a location of the wire, and a depressed portion is formed in a location of the gap, and the outer layer has a protrusion part in the depressed portion of the inner layer, the protrusion part entering deeper than the reinforcement layer through the gap and extending in an axis direction of the catheter.

Further, a length of the protrusion part of the outer layer in the axis direction of the catheter is longer than a length of the gap of the reinforcement layer in the axis direction of the catheter.

Embodiment 2 of the present invention provides a balloon catheter including the catheter according to embodiment 1, and a balloon joined to the outer layer, in which the outer layer has an uneven outer peripheral surface on which a protruded portion is formed in the location of the wire, and a depressed portion is formed in the location of the gap, the uneven outer peripheral surface of the outer layer being arranged along the outer peripheral surface of the inner layer, and the balloon enters into the depressed portion of the outer layer

### EFFECT OF THE INVENTION

In the catheter according to embodiment 1 of the present invention, the inner layer has an uneven outer peripheral surface on which a protruded portion is formed in a location of the wire, and a depressed portion is formed in a location of the gap between adjacent portions of the wire, and the outer layer has a protrusion part in the depressed portion of the inner layer, the protrusion part entering deeper than the reinforcement layer through the gap and extending in the axis direction of the catheter. Therefore, the joining strength between the inner layer and the outer layer can be improved, and the risk of detachment of the outer layer from the inner layer can be reduced due to the anchoring effect of the protrusion part of the outer layer caught in the reinforcement layer even when the outer layer is dragged in the axis direction (the distal end and proximal end directions) by a stenosis site or an obstructed segment.

Further, the length of the protrusion part of the outer layer in the axis direction of the catheter is longer than the length of the gap of the reinforcement layer in the axis direction of the catheter. Therefore, the anchoring effect between the protrusion part of the outer layer and the reinforcement layer can be enhanced not only in the axis direction but also in the radial direction, which in turn can further reduce the risk of detachment of the outer layer from the inner layer.

In the balloon catheter according to embodiment 2 of the present invention, the outer layer has an uneven outer peripheral surface on which a protruded portion is formed in a location of the wire, and a depressed portion is formed in a location of the gap, the uneven outer peripheral surface of the outer layer being arranged along the outer peripheral surface of the inner layer, and the balloon enters into the depressed portion of the outer layer. This configuration can enhance the joining strength between the balloon and the outer layer. Therefore, the risk of detachment of the balloon from outer layer can be reduced even when the balloon is inflated in the radial direction. Moreover, the configuration in which the balloon enters into the depressed portion of the outer layer can allow the thickness of the balloon to be thinner while maintaining the joining strength between the balloon and the outer layer, resulting in improved insertability of the balloon catheter into a blood vessel, bile duct, pancreatic duct, and the like.

### BRIEF DESCRIPTION OF THE DRAWINGS

Fig. 1 shows an overall view of the entire catheter according to the first example not being part of the invention.
Fig. 2 shows an enlarged view of section A in Fig. 1 .
Fig. 3 shows a cross-sectional view at the B-B cross-section of Fig. 2 .
Fig. 4 shows a Fig. 3 -equivalent cross-sectional view of a catheter according to the second example not being part of the invention.
Fig. 5 shows a Fig. 3 equivalent cross-sectional view of a catheter according to the third example not being part of the invention.
Fig. 6 shows a Fig. 2 -equivalent enlarged view of a catheter according to the fourth example not being part of the invention.
Fig. 7 shows a cross-sectional view at the C-C cross-section in Fig. 6 .
Fig. 8 shows a Fig. 3 -equivalent cross-sectional view of a catheter according to the first embodiment of the invention.
Fig. 9 shows an overall view of the entire catheter according to the second embodiment of the invention.
Fig. 10 shows an enlarged view of section D in Fig. 9 .
Fig. 11 shows a Fig. 10 -equivalent cross-sectional view of a balloon catheter according to the third embodiment of the invention.
Fig. 12 shows a Fig. 3 -equivalent cross-sectional view of a catheter according to the fifth example not being part of the invention.
Fig. 13 shows a Fig. 3 -equivalent cross-sectional view of a catheter according to the sixth example not being part of the invention.

### DESCRIPTION OF EMBODIMENTS

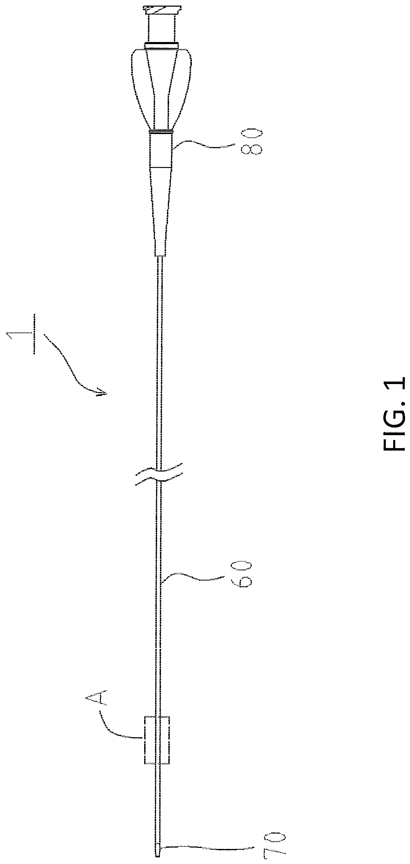

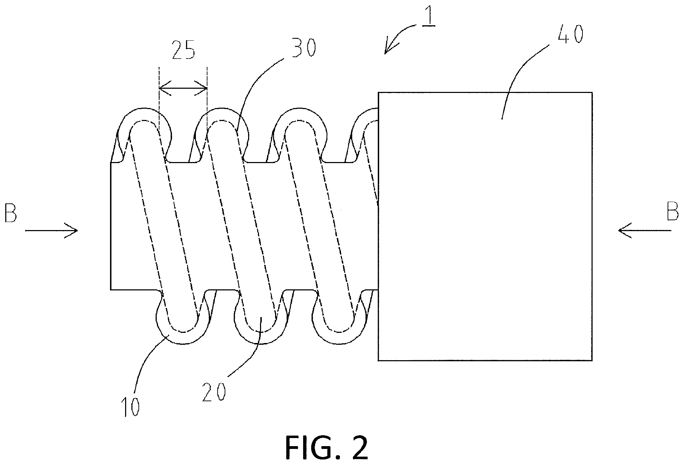

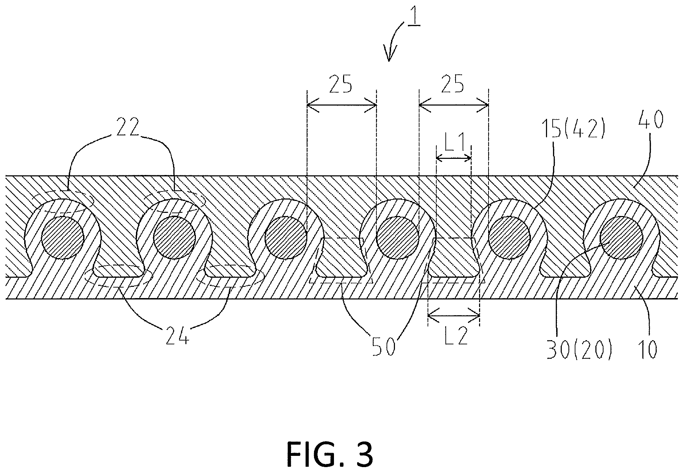

A catheter 1 according to the first example not being part of the invention will be described with reference to Figs. 1 to 3 . The left side in Fig. 1 corresponds to the distal end side (the front side) which is to be inserted into the body, and the right side corresponds to the proximal end side (the tail side) which is to be operated by an operator such as a physician. Fig. 2 shows an enlarged view of section A in Fig. 1 , and Fig. 3 shows a cross-sectional view of the B-B cross-section of Fig. 2 .

The catheter 1 may be used, for example, to diagnose or treat a stenosis site or an obstructed segment. As shown in Fig. 1 , the catheter 1 mainly includes a catheter shaft 60, a tip 70 joined to the distal end of the catheter shaft 60, and a connector 80 joined to the proximal end of the catheter shaft 60

As shown in Fig. 2 , the catheter shaft 60 includes, in the order of the radial direction from the inside, an inner layer 10; a reinforcement layer (coil body) 30 present inside the inner layer 10, the reinforcement layer (coil body) 30 having a wire 20 wound therearound so that a gap 25 is present between adjacent portions of the wire 20; and an outer layer 40 covering the reinforcement layer (coil body) 30. Note that a part of the outer layer 40 is not shown in Fig. 2 for better understanding.

The inner layer 10 is formed of a resin, through which a guide wire or another catheter can be inserted. There is no particular limitation on the resin material used for forming the inner layer 10, but PTFE (polytetrafluoroethylene) is used in the first example not being part of the invention.

The coil body 30 as a reinforcement layer is formed inside the inner layer 10. The coil body 30 is formed by winding the wire 20 in the clockwise direction toward the distal end side. Stainless steel (SUS304) is used as a material for the wire 20 of the coil body 30 in the first embodiment not being part of the invention, but the material is not limited to this. For example, metal materials such as tungsten and Ni-Ti alloys as well as resin materials such as reinforced plastics (PEEK) may be used. Note that the wire 20 of the coil body 30 may be wound in the counterclockwise direction toward the distal end side.

The outer layer 40 made of a resin is formed on the outer periphery of the coil body 30, and covers the inner layer 10 and the coil body 30. There is no particular limitation on the resin material for forming the outer layer 40, and polyamide, polyamide elastomer, polyester, polyurethane and the like may be used.

The tip 70 made of a resin is joined to the distal end of the catheter shaft 60 described above (see Fig. 1). There is no particular limitation on the resin for forming the tip 70, but it includes polyurethane, polyurethane elastomer, and the like. Further, the tip 70 may contain a radiopaque powder. As an example, when the tip 70 contains a radiopaque powder (for example, tungsten powder), in the range of between about 65 w% and about 90 w%, an operator such as a medical doctor can accurately detect the position of the catheter 1 under coronary angiography.

As shown in Fig. 3 , the inner layer 10 has an uneven outer peripheral surface 15 on which a protruded portion 22 is formed in a location of the wire 20, and a depressed portion 24 is formed in a location of the gap 25. Further, the outer layer 40 has a protrusion part 50 in the depressed portion 24 of the inner layer, the protrusion part 50 entering deeper than the reinforcement layer (coil body) 30 through the gap 25 and extending in an axis direction of the catheter 1.

The protrusion part 50 of the outer layer 40 is of an approximately trapezoidal shape. The length L2 of a lower base near the depressed portion 24 of the inner layer 10 is longer than the length L1 of an upper base near the center of the wire 20 (in other words, a location where the distance between the adjacent protruded portions 22 is the smallest) (L2 > L1). Further, the outer layer 40 has a corresponding uneven inner peripheral surface 42 along the uneven outer peripheral surface 15 of the inner layer 10.

According to the catheter 1, the outer layer 40 has the protrusion part 50 in a location of the depressed portion 24 of the inner layer 10, the protrusion part 50 entering deeper than the reinforcement layer (coil body) 30 through the gap 25 and extending in the axis direction of the catheter 1. This can improve the joining strength between the inner layer 10 and the outer layer 40. Further, the risk of detachment of the outer layer 40 from the inner layer 10 can be reduced by virtue of the anchoring effect in which the protrusion part 50 of the outer layer 40 is caught in the reinforcement layer (coil body) 30 even when the outer layer 40 is dragged in the axis direction (the distal end and proximal end directions) at a stenosis site or an obstructed segment upon insertion of the catheter 1 into a blood vessel, bile duct, pancreatic duct, and the like.

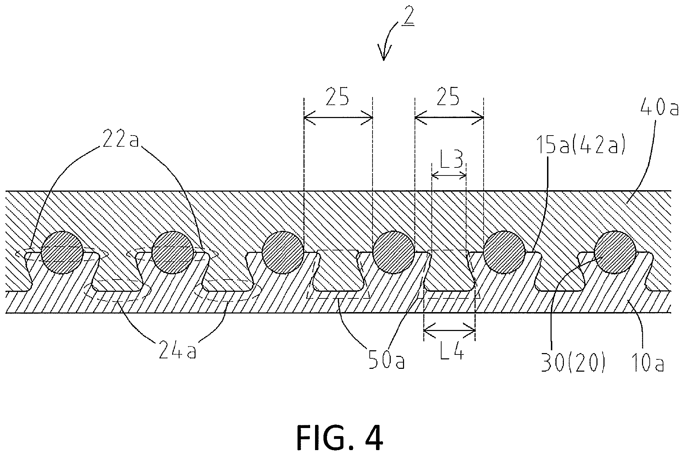

Next, a catheter 2 according to the second example not being part of the invention will be described with reference to Fig. 4 . Only differences from the catheter 1 shown in Fig. 3 will be described. In the catheter 2, the inner peripheral surface of the coil body 30 as a reinforcement layer is buried inside the inner layer 10a while the outer peripheral surface of the coil body 30 as a reinforcement layer is buried inside the outer layer 40a. The inner layer 10a has an uneven outer peripheral surface 15a on which a protruded portion 22a is formed in a location of the wire 20, and the depressed portion 24a is formed in a location of the gap 25. Further, the outer layer 40a has a protrusion part 50a in the depressed portion 24a of the inner layer 10a, the protrusion part 50a entering deeper than the reinforcement layer (coil body) 30 through the gap 25 and extending in the axis direction of the catheter 2.

The protrusion part 50a of the outer layer 40a is of an approximately trapezoidal shape as in the protrusion part 50 of the catheter 1. The length L4 of a lower base near the depressed portion 24a of the inner layer 10a is longer than the length L3 of an upper base near the center of the wire 20 (in other words, a location where the distance between the adjacent protruded portions 22a is the smallest) (L4 > L3). Further, the outer layer 40a has a corresponding uneven inner peripheral surface 42a along the uneven outer peripheral surface 15a of the inner layer 10a.

According to the catheter 2, the outer layer 40a has the protrusion part 50a in a location of the depressed portion 24a of the inner layer 10a, the protrusion part 50a entering deeper than the reinforcement layer (coil body) 30 through the gap 25 and extending in the axis direction as in the catheter 1. This can improve the joining strength between the inner layer 10a and the outer layer 40a. Further, the risk of detachment of the outer layer 40a from the inner layer 10a can be reduced by virtue of the anchoring effect in which the protrusion part 50a of the outer layer 40a is caught in the reinforcement layer (coil body) 30 even when the outer layer 40a is dragged in the axis direction (the distal end and proximal end directions) at a stenosis site or an obstructed segment upon insertion of the catheter 2 into a blood vessel, bile duct, pancreatic duct, and the like.

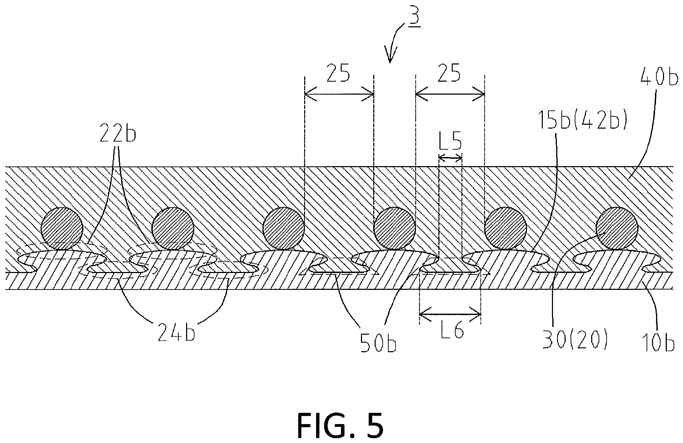

Next, a catheter 3 according to the third example not being part of the invention will be described with reference to Fig. 5 . Only differences from the catheter 1 shown in Fig. 3 will be described. In the catheter 3, the coil body 30 as a reinforcement layer is formed on the outer periphery of the inner layer 10b. The inner layer 10b has an uneven outer peripheral surface 15b on which a protruded portion 22b is formed in a location of the wire 20, and a depressed portion 24b is formed in a location of the gap 25. Further, the outer layer 40b has a protrusion part 50b in the depressed portion 24b of the inner layer 10b, the protrusion part 50b entering deeper than the reinforcement layer (coil body) 30 through the gap 25 and extending in the axis direction of the catheter 3.

The protrusion part 50b of the outer layer 40b is of an approximately trapezoidal shape as in the protrusion part 50 of the catheter 1. The length L6 of a lower base near the depressed portion 24b of the inner layer 10b is longer than the length L5 of an upper base at a location where the distance between the adjacent protruded portions 22b is the smallest (L6 > L5). Further, the outer layer 40b has a corresponding uneven inner peripheral surface 42b along the uneven outer peripheral surface 15b of the inner layer 10b.

According to the catheter 3, the outer layer 40b has the protrusion part 50b in a location of the depressed portion 24b of the inner layer 10b, the protrusion part 50b entering deeper than the reinforcement layer (coil body) 30 through the gap 25 and extending in the axis direction of the catheter 3. This can improve the joining strength between the inner layer 10b and the outer layer 40b. Further, the risk of detachment of the outer layer 40b from the inner layer 10b can be reduced by virtue of the anchoring effect in which the protrusion part 50b of the outer layer 40b is caught in the reinforcement layer (coil body) 30 even when the outer layer 40b is dragged in the axis direction (the distal end and proximal end directions) at a stenosis site or an obstructed segment upon insertion of the catheter 3 into a blood vessel, bile duct, pancreatic duct, and the like.

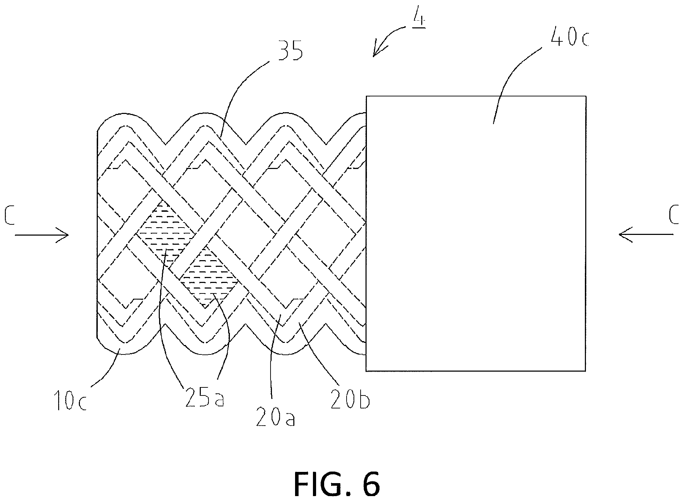

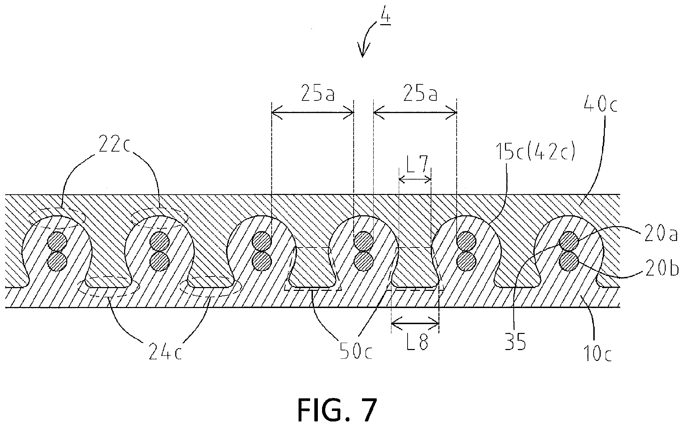

Next, a catheter 4 according to the fourth example not being part of the invention will be described with reference to Figs. 6 and 7 . Only differences from the catheter 1 shown in Figs. 2 and 3 will be described. The catheter 4 includes, in the order of the radial direction from the inside, an inner layer 10c; an reinforcement layer (braid) 35 present inside the inner layer 10, the reinforcement layer (braid) 35 having multiple wires 20a, 20b interwoven with each other so that a gap 25a is present between adjacent portions of the wires 20a, 20b; and an outer layer 40c covering the reinforcement layer (braid) 35 (see Fig. 6). Note that a part of the outer layer 40c is not shown in Fig. 6 for better understanding.

The reinforcement layer (braid) 35 includes a first wire 20a and a second wire 20b interwoven with each other in a web-like (mesh-like) fashion, in which the first wire 20a is wound in the clockwise direction toward the distal end side, and the second wire 20b is wound in the counterclockwise direction toward the distal end side. According to the fourth example not being part of the invention, a total of 16 wires (8 x 8) of 8 first wires 20a and 8 second wires 20b are interwoven with each other to form the reinforcement layer (braid) 35.

The first wires 20a and the second wires 20b of the reinforcement layer (braid) 35 may be made of the same material, or may be made of different materials. In the fourth embodiment not being part of the invention, first wires made of tungsten and second wires made of stainless steel (SUS 304) were used, but the materials are not particularly limited to these, and non-metal resin materials (for example, reinforced plastics) may be used.

As shown in Fig. 7 , the inner layer 10c has an uneven outer peripheral surface 15c on which a protruded portion 22c is formed in a location of the first wire 20a and the second wire 20b, and a depressed portion 24c is formed in a location of the gap 25a. Further, the outer layer 40c has a protrusion part 50c in the depressed portion 24c of the inner layer 10c, the protrusion part 50c entering deeper than the reinforcement layer (braid) 35 through the gap 25a and extending in the axis direction of the catheter 4.

The protrusion part 50c of the outer layer 40c is of an approximately trapezoidal shape. The length L8 of a lower base near the depressed portion 24c of the inner layer 10c is longer than the length L7 of an upper base near the centers of the wire 20a and the wire 20b of the reinforcement layer (braid) 35 (in other words, a location where the distance between the adjacent protruded portions 22c is the smallest) (L8 > L7). Further, the outer layer 40c has a corresponding uneven inner peripheral surface 42c along the uneven outer peripheral surface 15c of the inner layer 10c.

According to the catheter 4, the outer layer 40c has the protrusion part 50c in a location of the depressed portion 24c of the inner layer 10c, the protrusion part 50c entering deeper than the reinforcement layer (braid) 35 through the gap 25 and extending in the axis direction of the catheter 4. This can improve the joining strength between the inner layer 10c and the outer layer 40c. Further, the risk of detachment of the outer layer 40c from the inner layer 10c can be reduced by virtue of the anchoring effect in which the protrusion part 50c of the outer layer 40c is caught in the reinforcement layer (braid) 35 even when the outer layer 40c is dragged in the axis direction (the distal end and proximal end directions) at a stenosis site or an obstructed segment upon insertion of the catheter 4 into a blood vessel, bile duct, pancreatic duct, and the like.

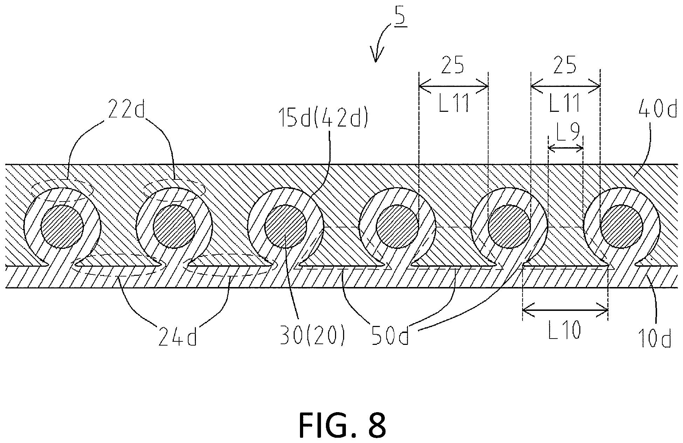

Next, a catheter 5 according to the first embodiment of the invention will be described with reference to Fig. 8 . Only differences from the catheter 1 shown in Fig. 3 will be described. In the catheter 5, an inner layer 10d has an uneven outer peripheral surface 15d on which a protruded portion 22d is formed in a location of the wire 20, and a depressed portion 24d is formed in a location of the gap 25. Further, the outer layer 40d has a protrusion part 50d in the depressed portion 24d of the inner layer 10d, the protrusion part 50d entering deeper than the reinforcement layer (coil body) 30 through the gap 25 and extending in the axis direction of the catheter 5.

The protrusion part 50d of the outer layer 40d is of an approximately trapezoidal shape as in the protrusion part 50 of the catheter 1. The length L10 of a lower base near the depressed portion 24d of the inner layer 10d (in other words, the largest length of the protrusion part 50d of the outer layer 40d in the axis direction) is longer than the length L9 of an upper base near the center of the wire 20 (in other words, a location where the distance between the adjacent protruded portions 22d is the smallest) (L10 > L9). Further, the length L10 of a lower base near the protrusion part 50d of the outer layer 40d (in other words, the largest length of the protrusion part 50d of the outer layer 40d in the axis direction) is longer than the length L11 of the gap 25 of the reinforcement layer (coil body) 30 in the axis direction of the catheter 5 (L10 > L11) (see Fig. 8). Further, the outer layer 40d has a corresponding uneven inner peripheral surface 42d along the uneven outer peripheral surface 15d of the inner layer 10d.

As described above, according to the catheter 5, the length L5 of a lower base of the protrusion part 50d of the outer layer 40d (in other words, the largest length of the protrusion part 50d of the outer layer 40d) is longer than the length L6 of the gap 25 of the reinforcement layer (coil body) 30 in the axis direction of the catheter 5. This can reduce the risk of detachment of the outer layer 40d from the inner layer 10d in the radial direction because the protrusion part 50d of the outer layer 40d is caught in the reinforcement layer (coil body) 30 even when the outer layer 40d is dragged outward in the radial direction. Thereby, the anchoring effect between the protrusion part 50d of the outer layer 40d and the reinforcement layer (coil body) 30 can be enhanced not only in the axis direction but also in the radial direction.

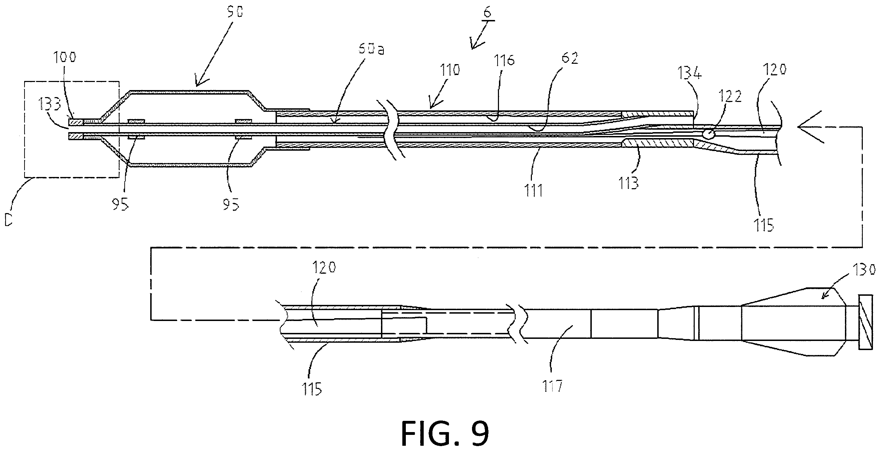

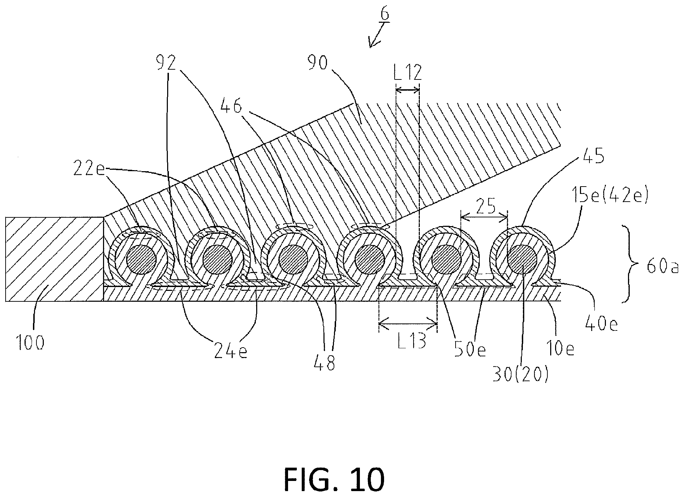

Next, a balloon catheter 6 according to the second embodiment of the invention will be described with reference to Figs. 9 and 10. Fig. 10 shows an enlarged view of section D in Fig. 9 . The balloon catheter 6 is used for expanding and treating, for example, a stenosis site or an obstructed segment.

As shown in Fig. 9, the balloon catheter 6 mainly includes a balloon 90, a tip 100, an outer shaft 110, an inner shaft 60a, a reinforcement body 120, and a connector 130.

The balloon 90 for expanding a stenosis site or obstructed segment includes a member made of a resin, the distal end of the balloon 90 is joined to the distal end of the inner shaft 60a and the tip 100, and the proximal end of the balloon 90 is joined to the distal end of the outer shaft 110.

The outer shaft 110 is a tubular member which constitutes an inflation lumen 116 for supplying a liquid such as contrast medium and physiological saline in order to inflate the balloon 90. The outer shaft 110 includes, in the order from the distal end side, a distal end outer shaft portion 111, a guide wire port portion 113, a middle outer shaft portion 115, and a proximal end outer shaft portion 117. The distal end outer shaft portion 111 and the middle outer shaft portion 115 are tubes made of a resin such as polyamide, polyamide elastomer, polyolefine, polyester, and polyester elastomer. The guide wire port portion 113 corresponds to a portion in which the distal end outer shaft portion 111, the middle outer shaft portion 115, and the inner shaft 60a are joined to each other.

The inner shaft 60a is inserted in the distal end outer shaft portion 111, and the inflation lumen 116 described above is formed between the distal end outer shaft portion 111 and the inner shaft 60a.

The proximal end outer shaft portion 117 is a metal tubular member which is referred to as a so-called hypotube. The distal end of the proximal end outer shaft portion 117 is inserted into and joined to the proximal end of the middle outer shaft portion 115. The connector 130 is attached to the proximal end of the proximal end outer shaft portion 117. When a liquid such as contrast medium and physiological saline for inflating the balloon 90 is supplied from an indeflator (not shown) which can be attached to the connector 130, the liquid is allowed to pass through the inflation lumen 116 to inflate the balloon 90. Note that there is no particular limitation on the material of the proximal end outer shaft portion 117, but stainless steel (SUS 302, SUS 304) and superelastic alloys such as Ni-Ti alloys may be used.

The inner shaft 60a forms a guide wire lumen 62 for inserting a guide wire thereinside. Further, the proximal end of the inner shaft 60a is joined to the guide wire port portion 113 of the outer shaft 110 to form a proximal end side guide wire port 134. Guide wires can be exchanged by an operator through the above proximal end side guide wire port 134.

The tip 100 is joined to the distal end of the inner shaft 60a and the distal end of the balloon 90. The tip 100 is made of a resin with plasticity. There is no particular limitation on the material, but polyurethane, polyurethane elastomer, and the like may be used. Further, the distal end tip 100 has a distal end side guide wire port 133 at the distal end.

The reinforcement body 120 is attached to the inner periphery of the distal end of the proximal end outer shaft portion 117. The reinforcement body 120 has a circular cross-section, and is a tapered metal wire member having a diameter decreasing toward the distal end. There is no particular limitation on the material of the reinforcement body 120, but stainless steel (SUS 304) and superelastic alloys such as Ni-Ti alloys may be used. The above reinforcement body 120 extends to the distal end outer shaft portion 111 through the middle outer shaft portion 115 and the guide wire port portion 113. Further, the reinforcement body 120 includes a pusher part 122 capable of abutting on the guide wire port portion 113.

Two markers 95 are attached to the outer periphery of the inner shaft 60a in the inside of the balloon 90. Thereby, an operator such as a physician can accurately detect the position of the balloon 90 under coronary angiography, facilitating reliable expansion of a stenosis site or obstructed segment.

As shown in Fig. 10 , the above inner shaft 60a includes, in the order of the radial direction from the inside, an inner layer 10e; a reinforcement layer (coil body) 30 present inside the inner layer 10e, the reinforcement layer (coil body) 30 having the wire 20 wound therearound so that the gap 25 is present between adjacent portions of the wire 20; and an outer layer 40e covering the reinforcement layer (coil body) 30.

The inner layer 10e has an uneven outer peripheral surface 15e on which a protruded portion 22e is formed in a location of the wire 20, and a depressed portion 24e is formed in a location of the gap 25. Further, the outer layer 40e has a protrusion part 50e in the depressed portion 24e of the inner layer 10e, the protrusion part 50e entering deeper than the reinforcement layer (coil body) 30 through the gap 25 and extending in the axis direction of the balloon catheter 6.

The protrusion part 50e of the outer layer 40e is of an approximately trapezoidal shape as in the protrusion part 50 of the catheter 1. The length L13 of a lower base near the depressed portion 24e of the inner layer 10e (in other words, the largest length of the protrusion part 50e of the outer layer 40e in the axis direction) is longer than the length L12 of an upper base near the center of the wire 20 (in other words, a location where the distance between the adjacent protruded portions 22e is the smallest) (L13 > L12). Further, the outer layer 40e has a corresponding uneven inner peripheral surface 42e along the uneven outer peripheral surface 15e of the inner layer 10e.

In the inner shaft 60a, the outer layer 40e has the protrusion part 50e in the depressed portion 24e of the inner layer 10e, the protrusion part 50e entering deeper than the reinforcement layer (coil body) 30 through the gap 25 and extending in the axis direction of the balloon catheter 6. This can improve the joining strength between the inner layer 10e and the outer layer 40e. Further, the risk of detachment of the outer layer 40e from the inner layer 10e can be reduced by virtue of the anchoring effect in which the protrusion part 50e of the outer layer 40e is caught in the reinforcement layer (coil body) 30 even when the outer layer 40e is dragged in the axis direction (the distal end and proximal end directions) upon insertion of the balloon catheter 6 into a blood vessel, bile duct, pancreatic duct, and the like.

Further, the outer layer 40e has an uneven outer peripheral surface 45 on which the protruded portion 46 is formed in a location of the wire 20, and the depressed portion 48 is formed in a location of the gap 25, the uneven outer peripheral surface 45 being arranged along the outer peripheral surface 15e of the inner layer 10e.

The balloon 90 enters into the depressed portion 48 of the outer layer 40e, and is joined to the outer peripheral surface 45 of the outer layer 40e. In other words, a protruding portion 92 is provided at the distal end of a balloon 90 so that the protruding portion 92 enters into the depressed portion 48 of the outer layer 40e.

The protruding portion 92 of the balloon 90 is joined to the depressed portion 48 of the outer layer 40e as described above. This configuration can enhance the joining strength between the balloon 90 and the outer layer 40e. Therefore, the risk of detachment of the balloon 90 from outer layer 40e can be reduced even when the balloon 90 is inflated in the radial direction. Further, the balloon 90 enters into the depressed portion 48 of the outer layer 40e. This configuration can allow the thickness of the balloon 90 to be thinner while maintaining the joining strength between the balloon 90 and the outer layer 40e, resulting in improved insertability of the balloon catheter 6 into a blood vessel, bile duct, pancreatic duct, and the like.

Note that the catheters 1 to 5 according to the first to fourth examples not being part of the invention and the first embodiment of the invention may be used as the inner shaft 60a of the balloon catheter 6 as shown in Figs. 9 and 10 .

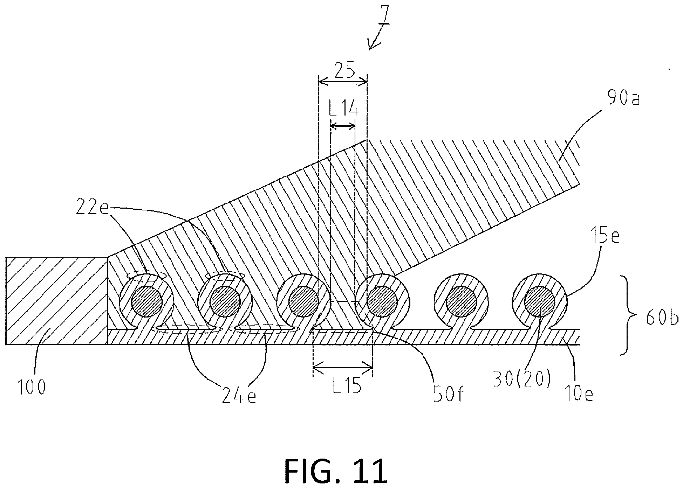

Further, the inner shaft 60a of the balloon catheter 6 includes the inner layer 10e, the reinforcement layer (coil body) 30, and the outer layer 40e as a cover in the radial order from the inside. However, the configuration is not limited to this. For example, as shown in Fig. 11 , in a balloon catheter 7 according to the third embodiment of the invention, an inner shaft 60b includes the inner layer 10e and the reinforcement layer (coil body) 30 present inside the inner layer 10e, the reinforcement layer (coil body) 30 having the wire 20 wound therearound so that the gap 25 is present between adjacent portions of the wire 20. In other words, unlike the balloon catheter 6, the inner shaft 60b does not have the outer layer 40e.

Only differences from the balloon catheter 6 shown in Fig. 10 will be described. In the balloon catheter 7, a balloon 90a enters into the depressed portion 24e of the inner layer 10e, and is joined to the outer peripheral surface 15e of the inner layer 10e. Specifically, the balloon 90a has a protrusion part 50f in the depressed portion 24e of the inner layer 10e, the protrusion part 50f entering deeper than the reinforcement layer (coil body) 30 through the gap 25 and extending in the axis direction of the balloon catheter 7

The protrusion part 50f of the balloon 90a is of an approximately trapezoidal shape. The length L15 of a lower base near the depressed portion 24e of the inner layer 10e is longer than the length L14 of an upper base near the center of the wire 20 (in other words, a location where the distance between the adjacent protruded portions 22 is the smallest) (L15 > L14).

In the balloon catheter 7, the balloon 90a has the protrusion part 50f in the depressed portion 24e of the inner layer 10e, the protrusion part 50f entering deeper than the reinforcement layer (coil body) 30 through the gap 25 and extending in the axis direction of the balloon catheter 7. This can improve the joining strength between the inner layer 10e and the balloon 90a. Further, the risk of detachment of the balloon 90a from the inner layer 10e can be reduced by virtue of the anchoring effect in which the protrusion part 50f of the balloon 90a is caught in the reinforcement layer (coil body) 30 even when the balloon 90a is dragged in the axis direction (the distal end and proximal end directions) at a stenosis site or an obstructed segment upon insertion of the balloon catheter 7 into a blood vessel, bile duct, pancreatic duct, and the like.

Further, the balloon 90 enters into the depressed portion 24e of the inner layer 10e. This configuration can allow the thickness of the balloon 90a to be thinner while maintaining the joining strength between the balloon 90a and the inner layer 10e, resulting in improved insertability of the balloon catheter 7 into a blood vessel, bile duct, pancreatic duct and the like.

Further, the protrusion parts 50, 50a, 50b, 50c, 50d, and 50e of the outer layers 40, 40a, 40b, 40c, 40d, and 40e; and the protrusion part 50f of the balloon 90a in the catheters 1 to 5 and the balloon catheters 6 and 7 as described above are shown to be of approximately trapezoidal shapes. However, they may be of any shape.

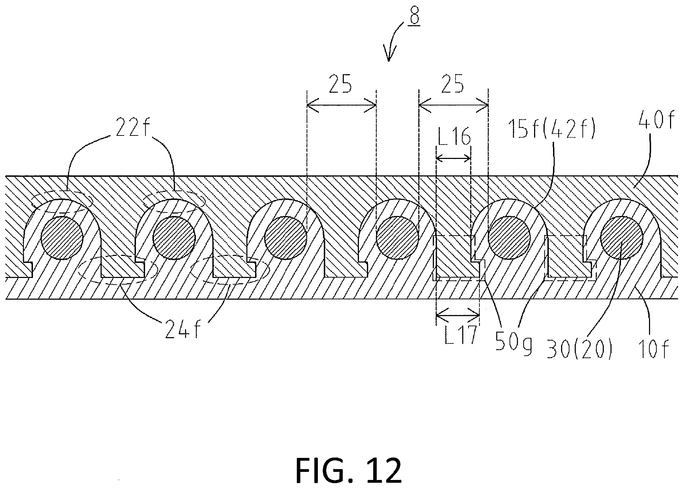

For example, in a catheter 8 according to the fifth example not being part of the invention as shown in Fig. 12 , an inner layer 10f has an uneven outer peripheral surface 15f on which a protruded portion 22f is formed in a location of the wire 20, and a depressed portion 24f is formed in a location of the gap 25. Further, the outer layer 40f has a protrusion part 50g in the depressed portion 24f of the inner layer 10f, the protrusion part 50g entering deeper than the reinforcement layer (coil body) 30 through the gap 25 and extending in the axis direction.

The protrusion part 50g of the outer layer 40f has an L-like shape. The length L17 of a lower base near the depressed portion 24f of the inner layer 10f is longer than the length L16 of an upper base near the center of the wire 20 (in other words, a location where the distance between the adjacent protruded portions 22f is the smallest) (L17 > L16). Further, the outer layer 40f has a corresponding uneven inner peripheral surface 42f along the uneven outer peripheral surface 15f of the inner layer 10f.

Note that in the catheter 8, the protrusion part 50g of the outer layer 40f extends only in the proximal end direction, but the configuration is not limited to this. It may be configured to extend only in the distal end direction of the catheter 8.

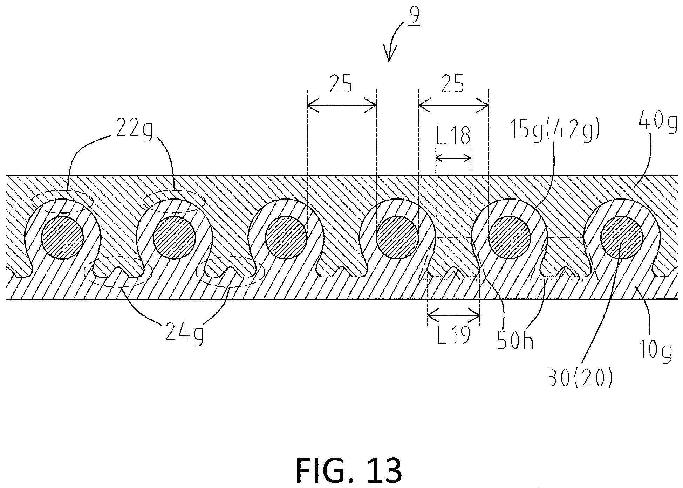

Further, in a catheter 9 according to the sixth example not being part of the invention as shown in Fig. 13 , an inner layer 10g has an uneven outer peripheral surface 15g on which a protruded portion 22g is formed in a location of the wire 20, and a depressed portion 24g is formed in a location of the gap 25. Further, an outer layer 40g has a protrusion part 50h in the depressed portion 24g of the inner layer 10g, the protrusion part 50h entering deeper than the reinforcement layer (coil body) 30 through the gap 25 and extending in the axis direction of the catheter 9.

The protrusion part 50h of the outer layer 40g bifurcates near the depressed portion 24g of the inner layer 10g. The length L19 of a lower base near the depressed portion 24g of the inner layer 10g is longer than the length L18 of an upper base near the center of the wire 20 (in other words, a location where the distance between the adjacent protruded portions 22g is the smallest) (L19 > L18). Further, the outer layer 40g has a corresponding uneven inner peripheral surface 42g along the uneven outer peripheral surface 15g of the inner layer 10g.

Further, the coil body 30 and the braid 35 are shown as examples of the reinforcement layer in the above descriptions, but the configuration is not limited to this. For example, a reinforcement layer in which a gap is included as a spiral slit provided in a hypotube (a metal tube) may be used in the catheters 1 to 5, 8, and 9 and the balloon catheters 6 and 7.

### DESCRIPTION OF SYMBOLS

1 to 5, 8, 9 Catheter
6, 7 Balloon catheter
10 to 10g Inner layer
15 to 15g Outer peripheral surface of inner layer
20, 20a, 20b Wire
22 to 22g Protruded portion (Inner layer)
24 to 24g Depressed portion (Inner layer)
30, 35 Reinforcement layer
40 to 40g Outer layer
42 to 42g Inner peripheral surface of outer layer
46 Protruded Portion (Outer Layer)
47 Depressed Portion (Outer Layer)
50 to 50h Protrusion part
60 Catheter shaft
60a, 60b Inner shaft
70, 100 Tip
80, 130 Connector
90, 90a Balloon
92 Protruding Portion
110 Outer shaft
120 Reinforcement layer

## Claims

1. A catheter comprising:
a tubular inner layer (10d; 10e);
a reinforcement layer (30) present inside the inner layer (10d; 10e) or on an outer periphery of the inner layer (10d; 10e), the reinforcement layer (30) having a wire (20) wound therearound so that a gap (25) is present between adjacent portions of the wire (20); and
an outer layer (40d; 40e) covering the reinforcement layer (30), wherein:
the inner layer (10d; 10e) has an uneven outer peripheral surface (15d; 15e) on which a protruded portion (22d; 22e) is formed in a location of the wire (20), and a depressed portion (24d; 24e) is formed in a location of the gap (25), and
the outer layer (40d; 40e) has a protrusion part (50d; 50e; 50f) in the depressed portion (24d; 24e) of the inner layer (10d; 10e), the protrusion part (50d; 50e; 50f) entering deeper than the reinforcement layer (30) through the gap (25) and extending in an axis direction of the catheter, **characterized in that**
a length of the protrusion part (50d; 50e; 50f) of the outer layer (40d; 40e) in the axis direction of the catheter is longer than a length of the gap (25) of the reinforcement layer (30) in the axis direction of the catheter.

2. A balloon catheter comprising
the catheter according to claim 1; and
a balloon (90; 90a) joined to the outer layer (40e), wherein:
the outer layer (40e) has an uneven outer peripheral surface (45) on which a protruded portion (46) is formed in the location of the wire (20), and a depressed portion (48) is formed in the location of the gap (25), the uneven outer peripheral surface (45) of the outer layer (40e) being arranged along the outer peripheral surface (15e) of the inner layer (10e), and
a protruding portion (92) is provided at the distal end of the balloon (90; 90a) so that the protruding portion (92) enters into the depressed portion (48) of the outer layer (40e).

## Patentansprüche

1. Katheter mit:
einer röhrenförmigen inneren Lage (10d; 10e);
einer in der inneren Lage (10d; 10e) oder am Außenumfang der inneren Lage (10d; 10e) vorhandenen Armierungslage (30) mit einem Draht (20), der so herumgewickelt ist, dass zwischen aufeinanderfolgenden Abschnitten des Drahts (20) ein Spalt (25) vorhanden ist; und
einer die Armierungslage (30) ummantelnden äußeren Lage (40d; 40e), wobei:
die innere Lage (10d; 10e) eine ungleichmäßige Außenumfangsfläche (15d; 15e) mit einem Vorsprung (22d; 22e) im Bereich des Drahts (20) und einer Aussparung (24d; 24e) im Bereich der Lücke (25) aufweist, und
die äußere Lage (40d; 40e) einen durch den Spalt (25) tiefer als die Armierungslage (30) eingedrungenen Vorsprung (50d; 50e; 50f) in der Aussparung (24d; 24e) der inneren Lage (10d; 10e) aufweist, der sich in Axialrichtung des Katheters erstreckt, **dadurch gekennzeichnet, dass**
die Länge des Vorsprung (50d; 50e; 50f) der äußeren Lage (40d; 40e) in Axialrichtung des Katheters größer ist als die Länge des Spalts (25) der Armierungslage (30) in Axialrichtung des Katheters.

2. Ballonkatheter mit
dem Katheter gemäß Anspruch 1; und
einem an die äußere Lage (40e) angefügten Ballon (90; 90a), wobei:
die äußere Lage (40e) eine ungleichmäßige Außenumfangsfläche (45) mit einem Vorsprung (46) im Bereich des Drahts (20) und einer Aussparung (48) im Bereich des Spalts (25) aufweist, wobei die ungleichmäßige Außenumfangsfläche (45) der äußeren Lage (40e) entlang der Außenumfangsfläche (15e) der inneren Lage (10e) angeordnet ist, und
an dem distalen Ende des Ballons (90; 90a) ein in die Aussparung der äußeren Lage (40e) eingedrungener Vorsprung (92) vorgesehen ist.

## Revendications

1. Cathéter comprenant :
une couche interne tubulaire (10d ; 10e) ;
une couche de renforcement (30) présente à l'intérieur de la couche interne (10d ; 10e) ou sur une périphérie externe de la couche interne (10d ; 10e), la couche de renforcement (30) ayant un fil (20) enroulé autour d'elle de sorte qu'un espace (25) est présent entre des parties adjacentes du fil (20) ; et
une couche externe (40d ; 40e) recouvrant la couche de renforcement (30), dans lequel :
la couche interne (10d ; 10e) a une surface périphérique externe irrégulière (15d ; 15e) sur laquelle une partie en saillie (22d ; 22e) est formée à un emplacement du fil (20), et une partie enfoncée (24d ; 24e) est formée à un emplacement de l'espace (25), et
la couche externe (40d ; 40e) a une partie de saillie (50d ; 50e ; 50f) dans la partie enfoncée (24d ; 24e) de la couche interne (10d ; 10e), la partie de saillie (50d ; 50e ; 50f) entrant plus profondément que la couche de renforcement (30) à travers l'espace (25) et s'étendant dans une direction d'axe du cathéter, **caractérisé en ce que**
une longueur de la partie de saillie (50d, 50e ; 50f) de la couche externe (40d ; 40e) dans la direction d'axe du cathéter est plus longue qu'une longueur de l'espace (25) de la couche de renforcement (30) dans la direction d'axe du cathéter.

2. Cathéter à ballonnet comprenant
le cathéter selon la revendication 1 ; et
un ballon (90 ; 90a) relié à la couche externe (40e), dans lequel :
la couche externe (40e) a une surface périphérique externe irrégulière (45) sur laquelle une partie en saillie (46) est formée à l'emplacement du fil (20), et une partie enfoncée (48) est formée à l'emplacement de l'espace (25), la surface périphérique externe irrégulière (45) de la couche externe (40e) étant disposée le long de la surface périphérique externe (15e) de la couche interne (10e), et
une partie saillante (92) est prévue à l'extrémité distale du ballon (90 ; 90a) de sorte que la partie saillante (92) pénètre dans la partie enfoncée (48) de la couche externe (40e).
